# EUROPÄISCHE PATENTSCHRIFT

(11) **EP 2 228 051 B1**
(45) Veröffentlichungstag und Bekanntmachung des Hinweises auf die Patenterteilung: **19.06.2013**
(21) Anmeldenummer: 10005710.8
(22) Anmeldetag: 09.11.2007
(51) Int. Cl.: A61K 8/60, A61K 8/06, A61K 8/25, A61K 8/26, A61K 8/44, A61K 8/73, A61K 8/88, A61Q 19/00, A61K 8/04, A61Q 17/04

(54) **Kosmetische Formulierung mit Glucosylglyceriden und Puderrohstoffen**
Cosmetic preparation containing glucosyl glycerides and powder
Formule cosmétique à glucosyle glycérides et matières premières en poudre

(30) Priorität: 17.11.2006 DE 102006055046
(43) Veröffentlichungstag der Anmeldung: 15.09.2010
(62) Teilanmeldung aus: 07120311.1
(73) Patentinhaber: Beiersdorf AG, 20253 Hamburg (DE)
(72) Erfinder: Kröpke, Rainer, 22869 Schenefeld (DE); Von der Fecht, Stephanie, 22880 Wedel (DE); Kruse, Inge, 20146 Hamburg (DE); Breitenbach, Ute, 22299 Hamburg (DE); Scherner, Cathrin, 22851 Norderstedt (DE); Knaupmeier, Stefanie, 32108 Bad Salzuflen (DE)

(56) Entgegenhaltungen:
- EP-A- 0 770 378
- WO-A-2005/030157
- WO-A-2006/122669
- WO-A2-99/37282
- DE-U1-202006 011 472
- M. KANEDA ET AL.: "Lilioside C, a Glycerol Glucoside from Lilium Lancifolium" PHYTOCHEMISTRY, Bd. 21, Nr. 4, 1982, Seiten 891-893, XP002597928

## Beschreibung

Die vorliegende Erfindung betrifft eine kosmetische Zubereitung mit Puderrohstoffen und ein oder mehreren Glucosylglyceriden.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Zu den in der Kosmetik eingesetzten Stoffen zählen u.a. Puderrohstoffe (beispielsweise Lauroyllysin, Nylon-12, Polymethylsilsesquioxan, Tapiokastärke, Natriumoctenylsuccinatstärke (INCI: sodium starch octenylsuccinate), Aluminiumoctenylsuccinatstärke (INCI: Aluminium starch octenylsuccinate), Distärkephosphat (INCI: Distarch Phosphate), Nylon 6/12, Mica, Silica, Talcum, mikrokristalline Cellulose, Kaolin. Puderrohstoffe werden beispielsweise dazu eingesetzt, überschüssiger Sebum der Haut zu absorbieren oder um das Hautgefühl der kosmetischen Zubereitung zu verbessern. Puderrohstoffe haben jedoch aufgrund ihres Quellverhaltens den Nachteil, die Viskosität der Formeln zu erhöhen. Die Rezepturen werden deutlich dickflüssiger und zäher im Fließverhalten. Diese Viskositätsanderung erschwert regelmaßig die Entnahme der Zubereitungen beispielsweise aus Quetschflaschen, wie sie gewöhnlich für Milche und Lotionen verwendet werden. Darüber hinaus sind derartige puderrohstoff-haltige Zubereitungen in der Regel nicht versprühbar.

Es war daher die Aufgabe der vorliegenden Erfindung die Nachteile des Standes der Technik zu beseitigen und Puderrohstoff-haltige Kosmetika mit verbessertem Vlskositätsvemalten zu entwickeln.

Überraschend gelöst wird die Aufgabe durch eine Kosmetische Zubereitung enthaltend eine Kombination aus
a) ein oder mehreren Puderrohstoffen und
b) ein oder mehreren Glucosylglyceriden, dadurch gekennzeichnet, dass die Zubereitung frei ist von (2-Hydroxyethyl)harnstoff, ausgenommen die folgenden Rezepturen:

| Substanz/Gew.-% | 1 | 2 | 3 | 4 | 5 | 6 |
|---|---|---|---|---|---|---|
| Glycerylstearatcitrat | 2 | 1,5 | 3 | | | |
| PEG-40-Stearate | | | | | | 2 |
| Glycerylstearat, selbstemulgierend | | | | 2 | | |
| Glycerylstearat | | | | | 2 | 2 |
| Cetylphosphat | | | | | 0,5 | |
| Wollwachsalkohol | | 0,5 | | 0,5 | 0,5 | |
| Stearinsäure | | | | 2 | 3 | |
| Myristylalkohol | | | | 2 | 3 | |
| Stearylalkohol | | 1 | | 0,5 | | 0,5 |
| Cetylalkohol | | | 3 | | | 2 |
| Cetostearylalkohol | 3 | 1 | 2 | 2 | | |
| Hydrierte Cocosfettglyceride | | 2 | | | 1 | |
| Butylen Glycol Dicaprylat/Dicaprat | | 1 | | | | |
| Ethyl Hexyl Cocoate | | 3 | | | | |
| Vaseline | | 4 | | | 2 | |
| Dicaprylylether | | 1 | 4 | | | |
| Myristylmyristat | 3 | | | | 1,5 | |
| Sheabutter | | | 2 | | | |
| C₁₂₋₁₅ Alkylbenzoate | | | 3 | 3 | 2 | 2 |
| Caprylsäure/Caprinsäure Triglyceride | 4 | | 4 | | | 1 |
| Octyldodecanol | 1 | | | | | |
| Paraffinum Liquidum | | | 1 | | | |
| Cyclomethicon | | | 1 | 3 | 1 | 3 |
| Dimethicon | 1 | | | 1 | | |
| Polydecen | | | | | 1 | |
| Dicaprylylcarbonat | | | | | | 2 |
| Erthylhexylmethoxycinnamat | | 3 | 2 | | 5 | 3 |
| TiO₂ | | | | 1 | 1 | |
| 2-Ethylhexyl-2-cyano-3-diphenylacrylat | | | | 5 | | |
| Bis-Ethylhexyloxypnenol-methoxyphenyltriazine | | 1 | | | | 1 |
| Ethylhexyltriazon | | | 2 | | | 2 |
| Butylmethoxydibenzoylmethan | | | 1 | | 1 | |
| Natrium-Ascorbylphosphat | | | | | 0,2 | |
| Kreatin | | | | 0,3 | | 0,5 |
| Kreatinin | | | | 0,1 | | 0,5 |
| Ubichinon (Q10) | | 0,05 | | 0,05 | | 0,02 |
| Taurin | | | 1,0 | | | |
| Phytinsäure | | | | 0,1 | | |
| Tocapherylacetat | | | 0,5 | | | |
| Weinsäure, Natriumsalz | | | | | | 0,1 |
| α-Glucosylrutin | 0,1 | | | | | |
| Zitronensäure, Natriumsalz | 0,1 | | | | | |
| Glycerylglucosid | 5 | 5 | 5 | 5 | 5 | 5 |
| Trinatrium EDTA | | | 0,2 | | 0,2 | |
| Iminodisuccinat | | 0,1 | | | | |
| Phenoxyethanol | | 0,3 | | | 0,8 | 0,4 |
| p-Hydroxybenzoesäurealkylester (Paraben) | | 0,5 | 0,4 | 0,3 | 0,4 | |
| Diazolidinylhamstoff | | 0,25 | | | | 0,2 |
| Hexamidindindiisethionat | | | | 0,04 | 0,05 | |
| 1,3-Dimethylol-5,5-dimethyl-hydantoin | | | 0,2 | | | |
| Iodopropynylbutylcarbamat | | 0,1 | | 0,05 | | |
| 2-Ethylhexylglycerinether | | | | | 3 | |
| Ethanol denaturiert | 3 | 1 | 2 | | | 8 |
| Ammoniumacryloyldimethyltaurat/VP Copolymer | 0,3 | 0,7 | 0,5 | 0,2 | 0,1 | 0,8 |
| Glycerin | 10 | 8 | 10 | 5 | 15 | 5 |
| Butylenglycol | | | 1 | 2 | | |
| Füllstoffe (Distärkephosphat, SiO₂, Talkum, Aluminiumstearat | 0,1 | 1 | 0,2 | 0,5 | 0,05 | 0,1 |
| Parfüm/Farbstoffe | q.s. | q.s | q.s. | q.s | q.s. | q.s. |
| Wasser | Ad 100 | Ad 100 | Ad 100 | Ad 100 | Ad. 100 | Ad. 100 |

Erfindungsgemäß vorteilhafte Ausführungsformen der vorliegenden Erfindung sind dadurch gekennzeichnet, dass die erfindungsgemäße Zubereitung das oder die Glucosylglyceride in einer Konzentration von 0,01 bis 10 Gewichts-%, bevorzugt in einer Konzentration von 0,05 bis 6 Gewichts-% und besonders bevorzugt In einer Konzentration von 0,1 bis 3 Gewichts%, jeweils bezogen auf das Gesamtgewicht der Zubereitung, enthält.

Vorteilhaft im Sinne der vorliegenden Erfindung sind Glucosylglyceride der Formel 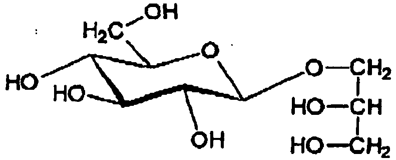 und/oder 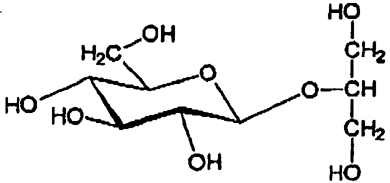 und/oder 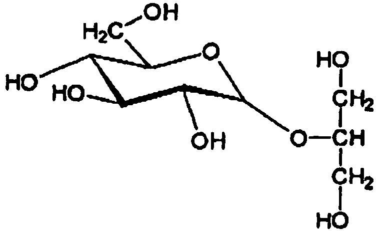 und/oder

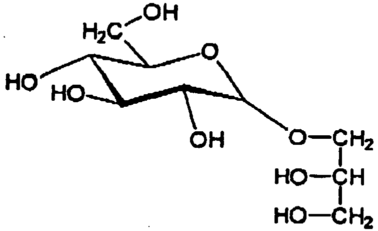

Zwar sind im Stand der Technik die DE 195 40 749 und die unveröffentlichten DE10 2005 023634, DE10 2005 023635, DE10 2005 023636, DE10 2005 023637, DE10 2005 023638, DE10 2005 023639, DE10 2005 023640, DE10 2005 023641, DE 10 2006 034530.4 und DE 20 2006 011472.6 beschrieben, sowie die JP 2004-331581, JP 2004-331576, JP 2004-331578, JP 2004-331579, JP 2004-331580, JP 2004-331582, JP 2004-331583, die hiermit als Referenz eingefügt sind, doch konnten diese Schriften nicht den Weg zur vorliegenden Erfindung weisen.

Darüber hinaus kennt der Stand der Technik die WO 99/37282, doch konnte diese Schrift ebenfalls nicht den Weg zur vorliegenden Erfindung weisen. Die WO 2006/122669 bildete als Stand der Technik nach Artikel 54(3) des Europäischen Patentübereinkommens die Grundlage für die aus dem Schutzbereich ausgenommenen Rezepturen.

Es ist erfindungsgemäß vorteilhaft, wenn die erfindungsgemäße Zubereitung Puderrohstoffe in einer Gesamtmenge von 0.01% bis Gewichts-10%, und bevorzugt in einer Konzentration von 0.1% bis Gewichts-1%, jeweils bezogen auf das Gesamtgewicht der Zubereitung enthalten.

Es ist erfindungsgemäß vorteilhaft, wenn die Puderrohstoffe eine mittlere Teilchengröße von 1 bis 100 µm, und erfindungsgemäß bevorzugt, wenn die Puderrohstoffe eine mittlere Teilchengröße von 10-50 µm ausweisen.

Die erfindungsgemäße Teilchengröße wird dabei mit dem folgenden Messverfahren bestimmt:
Laserlichtstreuung und/oder dynamische Lichtstreuung.

Es ist erfindungsgemäß vorteilhaft, wenn die erfindungsgemäßen Puderrohstoffe gewählt werden aus der Gruppe der Verbindungen Lauroyllysin, Nylon-12, Polymethylsilsesquioxen, Tapiokastärke, Natriumoctenylsuccinatstärke (INCI: sodium starch octenylsuccinate), Aluminiumoctenylsuccinatstärke (INCI: Aluminium starch octenylsuccinate), Distärkephosphat (INCI: Distarch Phosphate), Nylon 6/12, Mica, Silica, Talcum, mikrokristalline Cellulose, Kaolin.

Erfindungsgemäß vorteilhaft liegt die erfindungsgemäße Zubereitung in Form eines Gels, einer Emulsion oder einer Dispersion vor. Dabei ist es erfindungsgemäß bevorzugt, wenn die erfindungsgemäße Zubereitung in Form einer Emulsion vorliegt.

In einer erfindungsgemäß besonders bevorzugten Ausführungsform der vorliegenden Erfindung liegt die Zubereitung In Form einer O/W-Emulsion vor.

In diesem Falle ist es erfindungsgemäß bevorzugt, wenn die Zubereitung einen oder mehrere O/W-Emulgatoren gewählt aus der Gruppe der Verbindungen Glycerylstearatcitrat, Glycerylstearat, Cetearylglucoside, Stearinsäure sowie ihrer Salze, Polyglyceryl-3-methylglycosedistearat, Ceteareth-20, PEG-40 Stearat, PEG-100 Stearat, Natriumcetearylsulfat enthält.

Diese erfindungsgemäßen O/W-Emulgatoren können erfindungsgemäß vorteilhaft in einer Konzentration von 0,001 bis 10 Gewichts-% und bevorzugt in einer Konzentration von 0,1 bis 7 Gewichts-%, bezogen auf das Gesamtgewicht der Zubereitung in dieser enthalten sein.

Eine andere erfindungsgemäß bevorzugte Ausführungsform der vorliegenden Erfindung ist dadurch gekennzeichnet, dass die Zubereitung In Form einer W/O-Emulsion vorliegt.

Bei dieser Ausführungsform ist es erfindungsgemäß bevorzugt, wenn die Zubereitung einen oder mehrere W/O-Emulgatoren gewählt aus der Gruppe der Verbindungen Polyglyceryl-2-dipolyhydroxystearat, PEG-30 Dipolyhydroxystearat, Cetyl Dimethicon Copolyol, Polyglyceryl-3 Diisostearat enthält.

Diese erfindungsgemäßen W/O-Emulgatoren können erfindungsgemäß vorteilhaft in einer Konzentration von 0,1 bis 10 Gewichts-% und bevorzugt in einer Konzentration von 0,2 bis 7 Gewichts-%, bezogen auf das Gesamtgewicht der Zubereitung in dieser enthalten sein.

Erfindungsgemäß vorteilhafte Ausführungsformen der vorliegenden Erfindung sind dadurch gekennzeichnet, dass die Zubereitung einen oder mehrere weitere UV-Filter enthält, gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylenbis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan / Dimethylsiloxan - Copolymer; 2,4-Bis-{[4-(2-ethyl-hexyloxy)-2-hydroxy]-phenyl}-6-(4-methoxyphenyl)-1,3,5-triazin (INCI :Bis-Ethylhexyloxyphenol Methoxyphenyl Triazin); Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titiandioxid; Zinkoxid in einer Konzentration von 0,01 bis 40 Gewichts-% und bevorzugt in einer Konzentration von 1 bis 20 Gewichts-% bezogen auf das Gesamtgewicht der Zubereitung.

Es ist erfindungsgemäß vorteilhaft, wenn die erfindungsgemäße Zubereitung frei ist von p-Methylbenzylidencampher.

Die Pigmente (Titandioxid, Zinkoxid) können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente (Titandioxid, Zinkoxid) können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), Bariumsulfat (BaSO₄) oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß vorteilhafte Ausführungsformen der vorliegenden Erfindung sind dadurch gekennzeichnet, dass die erfindungsgemäße Zubereitung ein oder mehrere Verbindungen gewählt aus der Gruppe der Parabene, Phenoxyethanol, Ethylhexylglycerin, 2-Methylpropan-1,3-diol, Butylenglycol, Propylenglycol enthält, die in einer Gesamtkonzentration von 0,01 bis 10 Gewichts-%, bezogen auf das Gesamtgewicht der Zubereitung in dieser vorliegen können.

Erfindungsgemäß vorteilhaft enthält die erfindungsgemäße Zubereitung als weitere Inhaltsstoffe eine oder mehrere Verbindungen gewählt aus der Gruppe der Verbindungen Folsäure, Phytoen, D-Biotin, Coenzym Q10, alpha-Glucosylrutin, Carnitin, Harnstoff, Polydocanol, natürliche und/oder synthetische Isoflavonoide, insbesondere Genistein, Flavonoide, Carotinoide, Kreatin, Kreatinin, Taurin, Ascorbinsäure + Derivate, Sauerstoff, Tocopherol + Ester, Dihydroxyaceton; 8-Hexadecen-1,16-dicarbonsäure, lang- wie auch kurzkettige Hyaluronsäure (d.h. Hyaluronsäure mit einem mittleren Molekulargewicht von 1 Million bis 3 Million Dalton, wie auch 5000 Dalton - 1 Million Dalton) und/oder Licochalcon A. Derartige Inhaltsstoffe können jeweils in einer Einzelkonzentration von 0,01 bis 10 Gewichts-%, bezogen auf das Gesamtgewicht der Zubereitung, in dieser enthalten sein. Erfindungsgemäß besonders bevorzugt ist dabei der Einsatz von Carnitin.

Es ist erfindungsgemäß vorteilhaft, wenn die erfindungsgemäße Zubereitung zusätzlich Harnstoff in einer Konzentration von 0,01 bis 50 Gewichts-%, bevorzugt in einer Konzentration von 0.1 bis 20 Gewichts% und besonders bevorzugt in einer Konzentration von 1 bis 15 Gewichts% bezogen auf das Gesamtgewicht der Zubereitung, enthält.

Ferner vorteilhaft können die erfindungsgemäßen Zubereitungen auch Repellentien zum Schutz vor Mücken, Zecken und Spinnen und dergleichen enthalten. Vorteilhaft sind z. B. N,N-Diethyl-3-methylbenzamid (Handelsbezeichnung: "DEET"), Dimethylphtalat (Handelsbezeichnung: Palatinol M, DMP), 1-Piperidincarbonsäure-2-(2-hydroxyethyl)-1-methylpropylester sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Panthenol, Biosaccaride Gum-1, Glycine Soja, Ethylhexyloxyglycerin, Pyrrolidoncarbonsäure und Hyaluronsäure. Insbesondere vorteilhaft ist auch ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Es ist vorteilhaft im Sinne der vorliegenden Erfindung, wenn die erfindungsgemäße Zubereitung ein oder mehrere Feuchthaltemittel in einer Gesamtkonzentration von 0,1 bis 20 Gewichts-% und bevorzugt in einer Gesamtkonzentration von 0,5 bis 10 Gewichts-%, jeweils bezogen auf das Gesamtgewicht der Zubereitung, enthält.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, 2-Methylpropan-1,3-diol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Carragenanen, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCI: Ammonium Acryloyldimethyltaurate/VP Copolymer).

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP) 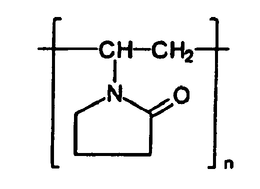 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol* CC bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*) und/oder Diethylhexylnaphthalat (*Hallbrite TQ oder Corapan TQ von Symrise)*.

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO₁,₅ und/oder R₃SiO₀,₅ und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
   wobei die verwendeten Mengenateile so gewählt werden, dass die Menge des Wasserstoffes des Organopolysiloxans (1) oder der ungesättigten aliphatischen Gruppen des Organopolysiloxans (2)

- im Bereich von 1 bis 20 mol-% liegt, wenn das Organopolysiloxan nicht zyklisch ist und
- im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische Wirkstoffe enthalten, wobei bevorzugte Wirkstoffe Antioxidantien sind, welche die Haut vor oxidativer Beanspruchung schützen können.

Erfindungsgemäße Rezepturen, welche z. B. bekannte Antifaltenwirkstoffe wie Flavonglycoside (insbesondere α-Glycosylrutin), Coenzym Q10, Retinol und Ester, Vitamin E und/oder Derivate und dergleichen enthalten, eignen sich insbesondere vorteilhaft zum Schutz vor ästhetisch unattraktiven Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen) und ermüdete Haut. Weiterhin vorteilhaft eignen sie sich gegen das Erscheinungsbild der trockenen bzw. rauhen Haut.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhaft im Sinne der vorliegenden Erfindung sind Zubereitungen zur Pflege der Haut: sie können dem kosmetischen Lichtschutz, ferner als Schminkprodukt in der dekorativen Kosmetik dienen.

Entsprechend ihrem Aufbau können kosmetische Zusammensetzungen im Sinne der vorliegenden Erfindung, beispielsweise verwendet werden als Hautschutzcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Es ist auch vorteilhaft im Sinne der vorliegenden Erfindung, kosmetische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische Zubereitungen, die in der Form eines Sonnenschutzmittels vorliegen.

Erfindungsgemäß ist insbesondere die Verwendung der erfindungsgemäßen Zubereitung zum Schutz vor Hautalterung (insbesondere zum Schutz vor UV-bedingter Hautalterung) sowie als Sonnenschutzmittel.

Erfindungsgemäß ist die Verwendung der erfindungsgemäßen Zubereitungen zur Verminderung und Verhinderung von Fältchen und Falten.

Erfindungsgemäß ist die Verwendung der erfindungsgemäßen Zubereitungen zur Reparatur der Haut (insbesondere von Hautschäden, die durch Waschstress hervorgerufen wurden). Erfindungsgemäß vorteilhaft weist die erfindungsgemäße Zubereitung einen pH-Wert von 5 bis 8 auf. Dieser kann durch die herkömmlichen Säuren, Basen und Puffersysteme eingestellt werden.

Zur Anwendung werden die erfindungsgemäßen kosmetischen Zubereitungen in der für Kosmetika üblichen Weise auf die Haut und/oder die Haare in ausreichender Menge aufgebracht.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### O/W-Lotion:

| **Produktbezeichnung** | **Menge [%]** | **Menge [%]** | **Menge [%]** |
|---|---|---|---|
| Glycerin | 6,00 | 8,00 | 12,00 |
| Cetyl Palmitate | 10,00 | 10,00 | 10,00 |
| Paraffinum Liquidum | 8,00 | 8,00 | 8,00 |
| Cetyl Alcohol | 3,00 | 2,00 | |
| Stearyl Alcohol | | 1,00 | 3,00 |
| Cyclomethicone | 3,00 | 3,00 | 3,00 |
| Sorbitan Stearate | 2,00 | 2,00 | 2,00 |
| Aluminum Starch Octenylsuccinate | 1,50 | 0,75 | 1,50 |
| Phenoxyethanol | 0,80 | 0,80 | 0,80 |
| Methylparaben | 0,30 | 0,30 | 0,40 |
| Carbomer | 0,25 | 0,25 | 0,25 |
| Propylparaben | 0,10 | 0,10 | |
| Sodium Hydroxide | 0,03 | 0,03 | 0,03 |
| Glucosylglyceride | 1,00 | 1,50 | 2,00 |
| Urea | 2,00 | 5,00 | 3,00 |
| Aqua | Ad 100, 00 | Ad 100,00 | Ad 100,00 |

### O/W-Creme 1:

| **Produktbezeichung** | **Menge [%]** | **Menge [%]** |
|---|---|---|
| Glycerin | 10,00 | 12,00 |
| Hydrogenated Coco-Glycerides | 5,00 | 5,00 |
| Stearyl Alcohol | 3,50 | 3,50 |
| Stearic Acid | 3,00 | 3,00 |
| Paraffinum Liquidum | 3,00 | 3,00 |
| Cetyl Alcohol | 1,50 | |
| Dimethicone | 1,00 | 1,00 |
| Phenoxyethanol | 0.80 | 0,80 |
| Glyceryl Stearate | 0,50 | 0,50 |
| Sorbitan Stearate | 0,50 | 0,50 |
| PEG-100 Stearate | 0,50 | 0,50 |
| Methylparaben | 0,40 | 0,40 |
| Carbomer | 0,20 | 0,20 |
| Distarch Phosphate | 1,00 | 0,20 |
| Mica | | |
| Glucosylglyceride | 1,50 | 2,00 |
| Urea | 5,00 | 3,00 |
| Hyaluronsäure | 0,50 | 0,30 |
| Propylparaben | 0,15 | 0,15 |
| Parfum | 0,03 | 0,03 |
| Aqua | Ad 100,00 | Ad 100,00 |

### 1. O/WEmulsionen:

| **Produktbezeichung** | **Menge [%]** | **Menge [%]** | **Menge [%]** | **Menge [%]** | **Menge [%]** | **Menge [%]** | **Menge [%]** |
|---|---|---|---|---|---|---|---|
| Glycerinmonostearat SE | 0,50 | 1,00 | 3,00 | | | 1,50 | |
| Glyceryl Stearat Citrat | 2,00 | | | 1,00 | 2,00 | | 2,50 |
| Stearinsäure | | 3,00 | 0,75 | 2,00 | | | |
| PEG-40 Stearat | 0,50 | | | | | 2,00 | |
| PEG-100 Stearat | | | 1,50 | | | | |
| Lauryl Methicon Copolyol | | | | 0,75 | | 0,50 | 0,50 |
| Cetyl Phosphat | | | 0,75 | | 1,00 | | |
| Stearyl Alkohol | | | 3,00 | | | 2,00 | 0,50 |
| Cetyl Alkohol | 2,50 | 1,00 | | | 0,50 | | 2,00 |
| UVASorb® K2A | 1,00 | | | 4,00 | | 5,00 | |
| Uvinul® A Plus | 3,00 | 2,50 | 0,50 | 0,25 | 1,00 | 0,50 | 4,00 |
| Bis-Ethylhexyloxyphenol Methoxyphenyl Triazin | | 1,00 | | | | 1,00 | 0,50 |
| Disodium Phenyl Dibenzimidazol Tetrasulfonat | | | | 1,00 | | 2,00 | |
| Ethylhexyl Triazon | 2,00 | | | 2,00 | | 2,00 | |
| Diethylhexyl Butamido Triazon | | 2,00 | | | | | |
| Ethylhexyl Methoxycinnamat | | 3,50 | | 10,00 | | | |
| Octocrylen | | | | 5,00 | 9,00 | 7,50 | 2,50 |
| Methylen Bis-Benztriazolyl Tetramethylbutylphenol | | | 2,00 | | 3,00 | | |
| Ethylhexylsalicylat | | | 3,00 | | | | 5,00 |
| Drometrizol Trisiloxan | | | 0,50 | | | 1,00 | |
| Titandioxid T 805 | | 1,50 | | | 1,00 | 0,50 | |
| Titandioxid MT-100Z | 1,00 | | | 3,00 | 1,00 | | |
| C₁₂₋₁₅ Alkyl Benzoat | | 2,50 | | | | 7,00 | 5,00 |
| Dicaprylylether | | | 3,50 | | 2,00 | | |
| Butylenglycol Dicaprylat/Dicaprat | 5,00 | | | 5,00 | 3,00 | | |
| Cetearyl Isononanoate | | 4,00 | | | | 2,00 | 2,00 |
| Dimethicon | | 0,50 | 1,00 | | 2,00 | | |
| Cyclomethicon | 2,00 | | | 4,50 | | | 0,50 |
| Dimethicon / Vinyl Dimethicon Crosspolymer | | 4,00 | | | | | 0,50 |
| PVP Hexadecen Copolymer | 0,50 | | | 0,50 | 1,00 | | 1,00 |
| Glycerin | 3,00 | 7,50 | | 7,50 | 5,00 | | 20,00 |
| Urea | | | | 5,00 | | 3,00 | |
| Hyaluronsäure | 0,10 | 0,30 | | | 0,50 | | 0,05 |
| Xanthangummi | 0,15 | | 0,05 | | | | 0,30 |
| Butylenglycol | 7,00 | 5,00 | | | | 7,00 | |
| Vitamin E Acetat | 0,50 | | 0,25 | 0,50 | 0,75 | | 1,00 |
| Tapiokastärke | 1,00 | 0,50 | 2,00 | 4,00 | 0,10 | 1,50 | 1,00 |
| Dioic Acid | 0,25 | | | 0,20 | | 0,25 | |
| Fucogel® 1000 | | | 1,50 | | | 5,00 | |
| Glucosylglyceride | 2,00 | 1,00 | 0,50 | 0,1 | 1,00 | 1,50 | 3,00 |
| DMDM Hydantoin | | 0,60 | 0,40 | 0,20 | | | |
| Methylparaben | 0,15 | | 0,25 | | 0,50 | | |
| Phenoxyethanol | 1,00 | 0,40 | | 0,40 | 0,50 | | 0,60 |
| EDTA | | 0,20 | 0,35 | 0,50 | 0,02 | | 0,03 |
| Ethanol | | 2,00 | 1,50 | | 3,00 | 5,00 | 1,00 |
| Insekt Repellent 3535 | | | 5,00 | | | | |
| Parfüm | 0,20 | 0,20 | | | | 0,30 | 0,40 |
| Wasser | ad 100 | ad 100 | ad 100 | ad 100 | ad 100 | ad 100 | ad 100 |

### Dünnflüssige bis sprühbare W/O-Emulsionen (zur Verwendung als Spray oder Aerosol):

| **Produktbezeichung** | **Menge [%]** | **Menge [%]** | **Menge [%]** | **Menge [%]** | **Menge [%]** |
|---|---|---|---|---|---|
| Cetyl Dimethicon Copolyol | 4,00 | | | 2,50 | 3,00 |
| Polyglyceryl-2 Dipolyhydroxystearat | | | 3,00 | | 1,00 |
| Isostearyl Diglyceryl Succinat | | | 0,75 | | 0,30 |
| Lauryl Methicon Copolyol | | | | 2,00 | |
| Polysorbat-65 | | | 2,00 | | 1,50 |
| PEG-100 Stearat | | | | 1,20 | 0,70 |
| Cetearyl Sulfat | | | 0,25 | | 1,00 |
| Dimethicon | | 4,00 | | | 2,00 |
| Cyclomethicon | 12,00 | 20,00 | | 30,00 | 15,00 |
| UVASorb® K2A | | | | 0,50 | |
| Uvinul® A Plus | 3,50 | 200 | 0,50 | 4,00 | 0,25 |
| Bis-Ethylhexyloxyphenol Methoxyphenyl Triazin | 1,00 | | | 0,50 | |
| Dinatrium Phenyl Dibenzimidazol Tetrasulfonat | | 1,50 | | | 2,00 |
| Drometrizol Trisiloxan | | | | 1,00 | |
| Ethylhexyl Methoxycinnamat | 3,00 | 4,00 | | | 10,00 |
| Ethylheyl Salicylat | | | 5,00 | | 3,50 |
| Octocrylen | | 5,00 | | 4,00 | |
| Diethylhexyl Butamido Triazon | | 1,00 | | | 6,50 |
| Ethylhexyl Triazon | 3,00 | | | | 4,00 |
| Titandioxid MT-100 Z | | 0,50 | 1,00 | 1,50 | 0,50 |
| Zinkoxid Z-Cote | 2,00 | | | | 4,00 |
| Dicaprylyl Carbonat | 5,00 | | 15,00 | | 4,00 |
| Dihexyl Carbonat | | 10,00 | | | |
| C12-15 Alkyl Benzoat | 7,00 | | 10,00 | | |
| Mineral Öl | 10,00 | | | | 6,00 |
| Cocoglyceride | | 2,00 | | 5,00 | |
| Distarch Phosphate | 0,10 | 0,50 | 0,30 | 1,00 | 0,40 |
| PVP Hexadecen Copolymer | | 0,75 | | | 0,40 |
| Glycerin | 5,00 | 12,50 | | 5,00 | 15,50 |
| Sorbitol | 5,00 | | 10,00 | | |
| Glucosylglyceride | 1,00 | 3,00 | 0,50 | 1,00 | 2,00 |
| α-Glucosylrutin | | | | | 0,15 |
| EDTA | | 0,15 | 0,03 | | 0,15 |
| Glycin Soja | 0,75 | | | 1,50 | |
| Magnesiumsulfat | 0,75 | 1,00 | | 0,45 | 1,00 |
| DMDM Hydantoin | | 0,05 | | | 0,10 |
| Phenoxyethanol | 1,00 | 0,75 | 0,50 | | 1,00 |
| Ethanol | 2,00 | | | 5,00 | 1,00 |
| Farbstoff, öllöslich | 0,02 | | | | |
| Parfüm | 0,30 | 0,45 | 0,35 | | 0,15 |
| Wasser | ad. 100 | ad. 100 | ad. 100 | ad. 100 | ad. 100 |

### W/O-Emulsionen (Cremes & Lotions):

| **Produktbezeichung** | **Menge [%]** | **Menge [%]** | **Menge [%]** | **Menge [%]** | **Menge [%]** |
|---|---|---|---|---|---|
| Cetyldimethicon Copolyol | | | | 4,00 | |
| Polyglyceryl-2-dipolyhydroxystearat | 5,00 | 4,50 | | | 4,50 |
| PEG-30-Dipolyhydroxystearat | | | 5,00 | 2,00 | |
| UVASorb® K2A | | | | 2,50 | |
| Uvinul ® A Plus | 3,00 | 1,00 | 0,50 | 0,25 | 2,50 |
| Phenylbenzmidazol Sulfonsäure | | 4,00 | | 2,00 | 0,50 |
| Ethylhexyl Methoxycinnamat | | 8,00 | | 5,00 | 4,00 |
| Diethylhexyl Butamido Triazon | 3,00 | 1,00 | | | 3,00 |
| Ethylhexyl Triazon | | | 3,00 | 4,00 | |
| Octocrylen | 7,00 | | 8,00 | | 2,50 |
| Drometrizol Trisiloxan | | | 3,00 | | |
| Titandioxid Uvinul® T 805 | 2,00 | 1,00 | | | |
| Titandioxid MT-100 TV | | | 3,00 | | 2,00 |
| Zinkoxid Z-Cote® HP1 | 2,50 | | 6,00 | | |
| Mineralöl | | | 10,0 | | 8,00 |
| Cocoglyceride | 4,00 | 6,50 | | | |
| C12-15 Alkyl Benzoate | | | | 9,00 | |
| Dicaprylylether | 10,00 | | | | 7,00 |
| Butylenglycol Dicaprylat/Dicaprat | | | 2,00 | 8,00 | 4,00 |
| Cyclomethicon | 2,00 | | | | 2,00 |
| PVP Eicosene Copolymer | 0,50 | | | 1,50 | 1,00 |
| Trinatrium EDTA | 1,00 | | | 0,35 | |
| Ethylhexyloxyglycerin | | 0,30 | 1,00 | | 0,50 |
| Methylpropandiol | | | | | 7,50 |
| Glycerin | 5,00 | 7,50 | | 7,50 | 2,50 |
| Glucosylglyceride | 0,50 | 2,50 | 1,50 | 0,50 | 1,00 |
| Butylenglycol | | 2,50 | 15,00 | | |
| Sodium starch octenylsuccinate | 1,00 | 0,50 | 0,25 | 2,00 | 0,10 |
| Glycin Soja | | 1,00 | 1,50 | | |
| MgSO₄ | 1,00 | 0,50 | | 0,50 | |
| Vitamin E | 0,50 | | 0,25 | | 1,00 |
| DMDM Hydantoin | | 0,60 | | 0,20 | |
| Methylparaben | 0,50 | | | 0,15 | |
| Phenoxyethanol | 0,50 | 0,40 | | 1,00 | 0,60 |
| Dihydroxiaceton | | | | 5,50 | |
| Ethanol | 3,00 | | 4,50 | | 1,00 |
| Parfüm | 0,20 | | 0,20 | | 0,20 |
| Wasser | ad 100 | ad 100 | ad 100 | ad 100 | ad 100 |

### Dünnflüssige bis sprühbare W/O-Emulsionen (zur Verwendung als Spray oder Aerosol):

| **Produktbezeichung** | **Menge [%]** | **Menge [%]** | **Menge [%]** | **Menge [%]** | **Menge [%]** |
|---|---|---|---|---|---|
| Cetyl Dimethicon Copolyol | 4,00 | | | 2,50 | 3,00 |
| Polyglyceryl-2 Dipolyhydroxystearat | | | 3,00 | | 1,00 |
| Isostearyl Diglyceryl Succinat | | | 0,75 | | 0,30 |
| Lauryl Methicon Copolyol | | | | 2,00 | |
| Polysorbat-65 | | | 2,00 | | 1,50 |
| PEG-100 Stearat | | | | 1,20 | 0,70 |
| Cetearyl Sulfat | | | 0,25 | | 1,00 |
| Dimethicon | | 4,00 | | | 2,00 |
| Cyclomethicon | 12,00 | 20,00 | | 30,00 | 15,00 |
| UVASorb® K2A | | | | 0,50 | |
| Uvinul® A Plus | 3,50 | 2,00 | 0,50 | 4,00 | 0,25 |
| Bis-Ethylhexyloxyphenol Methoxyphenyl Triazin | 1,00 | | | 0,50 | |
| Dinatrium Phenyl Dibenzimidazol Tetrasulfonat | | 1,50 | | | 2,00 |
| Drometrizol Trisiloxan | | | | 1,00 | |
| Ethylhexyl Methoxycinnamat | 3,00 | 4,00 | | | 10,00 |
| Ethylhexyl Salicylat | | | 5,00 | | 3,50 |
| Octocrylen | | 5,00 | | 4,00 | |
| Diethylhexyl Butamido Triazon | | 1,00 | | | 6,50 |
| Ethylhexyl Triazon | 3,00 | | | | 4,00 |
| Titandioxid MT-100 Z | | 0,50 | 1,00 | 1,50 | 0,50 |
| Zinkoxid Z-Cote | 2,00 | | | | 4,00 |
| Dicaprylyl Carbonat | 5,00 | | 15,00 | | 4,00 |
| Dihexyl Carbonat | | 10,00 | | | |
| C12-15 Alkyl Benzoat | 7,00 | | 10,00 | | |
| Mineral Öl | 10,00 | | | | 6,00 |
| Cocoglyceride | | 2,00 | | 5,00 | |
| Distarch Phosphate | 0,10 | 0,50 | 0,30 | 1,00 | 0,40 |
| PVP Hexadecen Copolymer | | 0,75 | | | 0,40 |
| Glycerin | 5,00 | 12,50 | | 5,00 | 15,50 |
| Sorbitol | 5,00 | | 10,00 | | |
| Glucosylglyceride | 1,00 | 3,00 | 0,50 | 1,00 | 2,00 |
| α-Glucosylrutin | | | | | 0,15 |
| EDTA | | 0,15 | 0,03 | | 0,15 |
| Glycin Soja | 0,75 | | | 1,50 | |
| Magnesiumsulfat | 0,75 | 1,00 | | 0,45 | 1,00 |
| DMDM Hydantoin | | 0,05 | | | 0,10 |
| Phenoxyethanol | 1,00 | 0,75 | 0,50 | | 1,00 |
| Ethanol | 2,00 | | | 5,00 | 1,00 |
| Farbstoff, öllöslich | 0,02 | | | | |
| Parfüm | 0,30 | 0,45 | 0,35 | | 0,15 |
| Wasser | ad.100 | ad.100 | ad.100 | ad.100 | ad. 100 |

### W/O-Emulsionen (Cremes & Lotions):

| **Produktbezeichnung** | **Menge** | **Menge** | **Menge** | **Menge** | **Menge** |
|---|---|---|---|---|---|
| | **[%]** | **[%]** | **[%]** | **[%]** | **[%]** |
| Cetyldimethicon Copolyol | | | | 4,00 | |
| Polyglyceryl-2-dipolyhydroxystearat | 5,00 | 4,50 | | | 4,50 |
| PEG-30-Dipolyhydroxystearat | | | 5,00 | 2,00 | |
| UVASorb® K2A | | | | 2,50 | |
| Uvinul ® A Plus | 3,00 | 1,00 | 0,50 | 0,25 | 2,50 |
| Phenylbenzmidazol Sulfonsäure | | 4,00 | | 2,00 | 0,50 |
| Ethylhexyl Methoxycinnamat | | 8,00 | | 5,00 | 4,00 |
| Diethylhexyl Butamido Triazon | 3,00 | 1,00 | | | 3,00 |
| Ethylhexyl Triazon | | | 3,00 | 4,00 | |
| Octocrylen | 7,00 | | 8,00 | | 2,50 |
| Drometrizol Trisiloxan | | | 3,00 | | |
| Titandioxid Uvinul® T 805 | 2,00 | 1,00 | | | |
| Titandioxid MT-100 TV | | | 3,00 | | 2,00 |
| Zinkoxid Z-Cote® HP1 | 2,50 | | 6,00 | | |
| Mineralöl | | | 10,0 | | 8,00 |
| Cocoglyceride | 4,00 | 6,50 | | | |
| C12-15 Alkyl Benzoate | | | | 9,00 | |
| Dicaprylylether | 10,00 | | | | 7,00 |
| Butylenglycol Dicaprylat/Dicaprat | | | 2,00 | 8,00 | 4,00 |
| Cyclomethicon | 2,00 | | | | 2,00 |
| PVP Eicosene Copolymer | 0,50 | | | 1,50 | 1,00 |
| Trinatrium EDTA | 1,00 | | | 0,35 | |
| Ethylhexyloxyglycerin | | 0,30 | 1,00 | | 0,50 |
| Methylpropandiol | | | | | 7,50 |
| Glycerin | 5,00 | 7,50 | | 7,50 | 2,50 |
| Glucosylglyceride | 0,50 | 2,50 | 1,50 | 0,50 | 1,00 |
| Butylenglycol | | 2,50 | 15,00 | | |
| Sodium starch octenylsuccinate | 1,00 | 0,50 | 0,25 | 2,00 | 0,10 |
| Glycin Soja | | 1,00 | 1,50 | | |
| MgSO₄ | 1,00 | 0,50 | | 0,50 | |
| Vitamin E | 0,50 | | 0,25 | | 1,00 |
| DMDM Hydantoin | | 0,60 | | 0,20 | |
| Methylparaben | 0,50 | | | 0,15 | |
| Phenoxyethanol | 0,50 | 0,40 | | 1,00 | 0,60 |
| Dihydroxiaceton | | | | 5,50 | |
| Ethanol | 3,00 | | 4,50 | | 1,00 |
| Parfüm | 0,20 | | 0,20 | | 0,20 |
| Wasser | ad 100 | ad 100 | ad 100 | ad 100 | ad 100 |

### Hydrodispersionen (zur Verwendung als Lotion oder Spray):

| **Produktbezeichung** | **Menge** | **Menge** | **Menge** | **Menge** | **Menge** |
|---|---|---|---|---|---|
| | **[%]** | **[%]** | **[%]** | **[%]** | **[%]** |
| PEG-40 Stearat | | 1,25 | | | |
| Cetyl Alkohol | | | | 2,00 | |
| Sodium Carbomer | | 0,20 | | 0,30 | |
| Acrylates/C10-30 Alkyl Acrylate Crosspolymer | | | 0,40 | 0,10 | 0,10 |
| Xanthan Gummi | 0,50 | 0,30 | 0,15 | | 0,50 |
| Dimethicon / Vinyl Dimethicon Crosspolymer | | | 5,00 | | 3,00 |
| UVASorb® K2A | | | | 3,50 | |
| Uvinul® A Plus | 0,25 | 3,50 | 0,50 | 2,00 | 1,50 |
| Bis-Ethylhexyloxyphenol Methoxypheny Triazin | | | 0,25 | | |
| Terephthaliden Dicampher Sulfonsäure | | 0,20 | | | 0,50 |
| Dinatrium Phenyl Dibenzimidazol Tetrasulfonat | 0,75 | | | | 1,00 |
| Ethylhexyl Methoxycinnamat | | | | 5,00 | 8,00 |
| Methylen Bis-Benzotriazolyl Tetramethylbutylphenol | | 1,00 | | | |
| Diethylhexyl Butamido Triazon | | | 2,00 | | |
| Ethylhexyl Triazon | 4,00 | | | 4,00 | |
| Octocrylen | | 4,00 | 10,00 | | 2,50 |
| Titandioxid MT-100 Z | 0,50 | | 2,00 | 3,00 | 1,00 |
| C₁₂₋₁₅ Alkyl Benzoate | 2,00 | 2,50 | | | |
| Butylenglycol Dicapryla/Dicaprat | 4,00 | | | 6,00 | |
| Dicaprylyl Carbonat | | 3,00 | | | |
| Cyclomethicon | | | 7,50 | | |
| Glucosylglyceride | 2,50 | 3,00 | 0,50 | 1,00 | 1,00 |
| Lanolin | | | | 0,35 | |
| Talcum | 1,00 | 0,10 | 0,75 | 1,50 | 0,30 |
| PVP Hexadecen Copolymer | 0,50 | | | 0,50 | 1,00 |
| Ethylhexyloxyglycerin | | 0,50 | 1,00 | | 0,50 |
| Glycerin | 10,00 | 7,50 | | 5,00 | 15,00 |
| Glycin Soja | | 1,50 | 1,00 | | |
| Vitamin E Acetat | 0,50 | 0,20 | 0,25 | 0,75 | 1,00 |
| α - Glycosylrutin | | 0,30 | | 0,25 | |
| Trinatrium EDTA | | 0,30 | 0,10 | 0,20 | |
| Konkaben LMB ® | 0,20 | | | | 0,15 |
| Methylparaben | 0,50 | | | 0,15 | |
| Phenoxyethanol | 0,50 | | | 1,00 | 0,60 |
| Ethanol | 3,00 | 7,00 | 3,50 | | 1,00 |
| Parfüm, Farbstoffe | q.s. | q.s. | q.s. | q.s. | q.s. |
| Wasser | ad 100 | ad 100 | ad 100 | ad 100 | ad 100 |

### Ölgele:

| **Produktbezeichnung** | **Menge** | **Menge** | **Menge** | **Menge** | **Menge** |
|---|---|---|---|---|---|
| | **[%]** | **[%]** | **[%]** | **[%]** | **[%]** |
| Octyldodecanol | 9,00 | 9,00 | | | |
| Caprylic/Capric Triglycerid | 9,00 | | 6,00 | | |
| C₁₂₋₁₅- Alkyl Benzoate | | | | 5,00 | 8,00 |
| Butylenglycol Dicaprylat/Dicaprat | | 9,00 | | | 8,00 |
| Dicaprylylether | 9,00 | | | 4,00 | |
| Dicaprylylcarbonat | | 7,00 | | | |
| Ethyl Galaktomannan (N-Hance® AG 200) | 3,50 | | | | 4,00 |
| C₂₀₋₄₀ Fettsäuren + Polyethylen (Performacid®350) | | | | 3,60 | |
| Glucosylglyceride | 2,50 | 3,00 | 1,00 | 1,00 | 0,50 |
| Hydroxyoctacosanyl Hydroxystearat | 2,00 | | | | |
| Disteardimonium Hectorit | 1,00 | | | | 1,00 |
| Cetyl Dimethicon | 0,50 | | 4,50 | | |
| Cyclomethicon | | | 15,00 | | 5,00 |
| UVASorb® K2A | | | | | 1,00 |
| Uvinul®A Plus | 1,00 | 3,50 | 2,75 | 2,00 | 0,50 |
| Ethylhexylmethoxycinnamat | 6,00 | | | 10,00 | 3,0 |
| Octocrylen | 3,50 | | 7,50 | 10,00 | |
| Ethylhexylsalicylat | | 3,50 | | | 4,00 |
| Ethylhexyl Triazon | | | 2,00 | | |
| Diethylhexyl Butamido Triazon | | 0,50 | | 3,00 | 4,0 |
| Nylon-6/12 | 1,50 | | 2,00 | | 3,00 |
| Kaolin | | 0,50 | | 0,75 | |
| Phenoxyethanol | 0,50 | | | | |
| Parfüm, Farbstoff | q.s. | q.s. | q.s. | q.s. | q.s. |
| Mineralöl | ad.100 | ad.100 | ad. 100 | | |
| Reisöl | | | | ad. 100 | ad.100 |

## Patentansprüche

1. Kosmetische Zubereitung enthaltend eine Kombination aus
a) ein oder mehreren Puderrohstoffen gewählt aus der Gruppe Tapiokastarke, Natriumoctenylsuccinatstärke, Aluminiumoctenylsuccinatstärke, Distärkephosphat und
b) ein oder mehreren Glucosylglyceriden,
**dadurch gekennzeichnet, dass** die Zubereitung frei ist von (2-Hydroxyethyl)hamstoff, ausgenommen die folgenden Rezepturen:
| Substanz/Gew.-% | **1** | **2** | **3** | **4** | **5** | **6** |
|---|---|---|---|---|---|---|
| Glycerylstearatecitrat | 2 | 1,5 | 3 | | | |
| PEG-40-Stearate | | | | | | 2 |
| Glycerylstearat, selbstemulgierend | | | | 2 | | |
| Glycerylstearat | | | | | 2 | 2 |
| Cetylphosphat | | | | | 0,5 | |
| Woilwachsalkohol | | 0,5 | | 0.5 | 0.5 | |
| Stearinsäure | | | | 2 | 3 | |
| Myristylalkohol | | | | 2 | 3 | |
| Stearylalkohol | | 1 | | 0.5 | | 0.5 |
| Cetylalkohol | | | 3 | | | 2 |
| Cetostearylalkohol | 3 | 1 | | 2 | | |
| Hydrierte Cocosfettglyceride | | 2 | | | 1 | |
| Butylen Glycol Dicaprylat/Dicaprat | | 1 | | | | |
| Ethyl Hexyl Cocoate | | 3 | | | | |
| Vaseline | | 4 | | | 2 | |
| Dicaprylylether | | 1 | 4 | | | |
| Myristylmyristat | 3 | | | | 1.5 | |
| Sheabutter | | | 2 | | | |
| C₁₂₋₁₅ Alkylbenzoate | | | 3 | 3 | | 2 |
| Caprylsäure/Caprinsäure Triglyceride | 4 | | 4 | | | 1 |
| Octyldodecanol | 1 | | | | | |
| Paraffinum Liquidum | | | 1 | | | |
| Cyclomethicon | | | 1 | 3 | 1 | 3 |
| Dimethicon | 1 | | | 1 | | |
| Polydecen | | | | | 1 | |
| Dicaprylylcarbonat | | | | | | 2 |
| Erthylhexylmethoxycinnamat | | 3 | 2 | | 5 | 3 |
| TiO₂ | | | | 1 | 1 | |
| 2-Ethylhexyl-2-Cyano-3-diphenylacrylat | | | | 5 | | |
| Bis-Ethylhexyloxyphenol-methoxyphenyltriazine | | 1 | | | | 1 |
| Ethylhexyltriazon | | | 2 | | | 2 |
| Butylmethoxydibenzoylmethan | | | 1 | | 1 | |
| Natrium-Ascorbylphosphat | | | | | 0,2 | |
| Kreatin | | | | 0,3 | | 0,5 |
| Kreatinin | | | | 0,1 | | 0.5 |
| Ubichinon (Q10) | | 0,05 | | 0,05 | | 0,02 |
| Taurin | | | 1,0 | | | |
| Phytinsäure | | | | 0.1 | | |
| Tocopherylacetat | | | 0,5 | | | |
| Welnsäure, Natriumsalz | | | | | | 0,1 |
| α-Glucosylrutin | 0,1 | | | | | |
| Citronensäure, Natriumsalz | 0,1 | | | | | |
| Glycerylglucosid | 5 | 5 | 5 | 5 | 5 | 5 |
| Trinatrium EDTA | | | 0,2 | | 0,2 | |
| Iminodisuccinat | | 0,1 | | | | |
| Phenoxyethanol | | 0,3 | | | 0,8 | 0,4 |
| p-Hydroxybenzoesäurealkylester (Paraben) | | 0,5 | 0,4 | 0,3 | 0,4 | |
| Diazolidinylharnstoff | | 0,25 | | | | 0,2 |
| Hexamidindiisethionat | | | | 0,04 | 0,05 | |
| 1,3-Dimethylol-5,5-dimethyl-hydantoin | | | 0,2 | | | |
| Iodopropynylbutylcarbamat | | 0,1 | | 0,05 | | |
| 2-Ethylhexylglycerinether | | | | | 3 | |
| Ethanol denaturiert | 3 | 1 | 2 | | | 8 |
| Ammoniumacryloyldimethyltaurat/VP Copolymer | 0,3 | 0,7 | 0,5 | 0,2 | 0,1 | 0,8 |
| Glycerin | 10 | 8 | 10 | 5 | 15 | 5 |
| Butylenglycol | | | 1 | 2 | | |
| Füllstoffe (Distärkephosphat, SIO₂, Talkum, Aluminiumstearat | 0,1 | 1 | 0,2 | 0,5 | 0,05 | 0,1 |
| Parfüm/Farbstoffe | q.s. | q.s | q.s. | q.s | q.s. | q.s. |
| Wasser | Ad 100 | Ad 100 | Ad 100 | Ad 100 | Ad. 100 | Ad. 100 |

2. Zubereitung nach Anspruch 1, **dadurch gekennzeichnet, dass** die Zubereitung Glucosylglyceride in einer Gesamtmenge von 0.01 bis 10 Gewichts-%, bezogen auf das Gesamtgewicht der Zubereitung enthalten.

3. Zubereitung nach einem der vorhergehenden Anspruche, **dadurch gekennzeichnet, dass** die Zubereitung Puderrohstoffe in einer Gesamtmenge von 0.01 bis 10 Gewichts-%, bezogen auf das Gesamtgewicht der Zubereitung enthalten.

4. Zubereitung nach einem der vorhergehenden Ansprüche, **dadurch gekennzeichnet, dass** die Puderrohstoffe eine mittlere Teilchengröße von 1 bis 100 µm aufweisen.

5. Kosmetische Zubereitung nach einem der vorhergehenden Ansprüche, **dadurch gekennzeichnet, dass** die Zubereitung in Form eines Gels, einer Emulsion oder einer Dispersion vorliegt.

6. Kosmetische Zubereitung nach einem der vorhergehenden Ansprüche, **dadurch gekennzeichnet, dass** die Zubereitung einen oder mehrere UV-Filter gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)-benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsiryl)oxy]disiloxanyl]propyl]-phenol; 3-(4-Methylbenzyliden)campher;3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäureamylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan / Dimethylsiloxan - Copolymer; 2,4-Bis-{[4-(2-ethyl-hexyloxy)-2-hydroxy]-phenyl}-6-(4-methoxyphenyl)-1,3,5-triazin (INCl :Bis-Ethylhexyloxyphenol Methoxyphenyl Triazin); Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titiandioxid; Zinkoxid in einer Konzentration von 0,01 bis 40 Gewichts-% bezogen auf das Gesamtgewicht der Zubereitung, enthält.

7. Kosmetische Zubereitung nach einem der vorhergehenden Anspruche, **dadurch gekennzeichnet, dass** die Zubereitung als weitere Inhaltsstoffe eine oder mehrere Verbindungen gewählt aus der Gruppe Folsäure, D-Biotin, Coenzym Q10, alpha-Glucosylrutin, Carnitin, natürliche und/oder synthetische Isoflavonoide, Genistein, Flavonoide, Carotinoide, Kreatin, Kreatinin, Taurin, Harnstoff, Ascorbinsäure und Derivate, Sauerstoff, Tocopherol + Ester, Dihydroxyaceton; lang- wie auch kurzkettige Hyaluronsäure (d.h. Hyaluronsäure mit einem mittleren Molekulargewicht von 1 Million bis 3 Million Dalton, wie auch 5000 Dalton -1 Million Dalton); 8-Hexadecen-1,16-dicarbonsäure und/oder Licochalcon A enthält.

8. Kosmetische Zubereitung nach einem der vorhergehenden Ansprüche, **dadurch gekennzeichnet, dass** die Zubereitung in Form einer O/W-Emulsion vorliegt.

9. Kosmetische Zubereitung nach einem der vorhergehenden Anspruche, **dadurch gekennzeichnet, dass** die die Zubereitung einen oder mehrere O/W-Emulgatoren gewählt aus der Gruppe der Verbindungen Glycerylstearatcitrat, Glycerylstearat, Cetearylglucoside, Stearinsäure sowie ihrer Salze, Polyglyceryl-3-methylglycosedistearat, Ceteareth-20, PEG-40 Stearat, PEG-100 Stearat, Natriumcetearylsulfat enthält.

10. Kosmetische Zubereitung nach einem der vorhergehenden Ansprüche, **dadurch gekennzeichnet, dass** die erfindungsgemäβe Zubereitung ein oder mehrere Verbindungen gewählt aus der Gruppe der Parabene, Phenoxyethanol, Ethylhexylglycerin, 2-Methylpropan-1,3-diol, Butylenglycol, Propylenglycol enthält.

## Claims

1. Cosmetic preparation comprising a combination of
a) one or more powder raw materials selected from the group tapioca starch, sodium octenylsuccinate starch, aluminium octenylsuccinate starch, distarch phosphate and
b) one or more glucosyl glycerides,
**characterized in that** the preparation is free from (2-hydroxyathyl)urea, with the exception of the following formulations:
| Substance/% by weight | 1 | 2 | 3 | 4 | 5 | 6 |
|---|---|---|---|---|---|---|
| Glyceryl stearate citrate | 2 | 1.5 | 3 | | | |
| PEG-40 stearate | | | | | | 2 |
| Glyceryl stearate, self-emulsifying | | | | 2 | | |
| Glyceryl stearate | | | | | 2 | 2 |
| Cetyl phosphate | | | | | 0.5 | |
| Wool wax alcohol | | 0.5 | | 0.5 | 0.5 | |
| Stearic acid | | | | 2 | 3 | |
| Myristyl alcohol | | | | 2 | 3 | |
| Stearyl alcohol | | 1 | | 0.5 | | 0.5 |
| Cetyl alcohol | | | 3 | | | 2 |
| Cetostearyl alcohol | 3 | 1 | | 2 | | |
| Hydrogenated coconut fatty glycerides | | 2 | | | 1 | |
| Butylene glycol dicaprylate/dicaprate | | 1 | | | | |
| Ethyl hexyl cocoate | | 3 | | | | |
| Vaseline | | 4 | | | 2 | |
| Dicaprylyl ether | | 1 | 4 | | | |
| Myristyl myristate | 3 | | | | 1.5 | |
| Shea butter | | | 2 | | | |
| C₁₂₋₁₅ alkyl benzoates | | | 3 | 3 | | 2 |
| Caprylic acid/capric acid triglycerides | 4 | | 4 | | | 1 |
| Octyldodecanol | 1 | | | | | |
| Paraffinum Liquidum | | | 1 | | | |
| Cyclomethicone | | | 1 | 3 | 1 | 3 |
| Dimethicone | 1 | | | 1 | | |
| Polydecene | | | | | 1 | |
| Dicaprylyl carbonate | | | | | | 2 |
| Ethylhexyl methoxycinnamate | | 3 | 2 | | 5 | 3 |
| TiO₂ | | | | 1 | 1 | |
| 2-Ethylhexyl 2-cyano-3-diphenylacrylate | | | | 5 | | |
| Bis-Ethylhexyloxyphenol methoxyphenyltriazine | | 1 | | | | 1 |
| Ethylhexyltriazone | | | 2 | | | 2 |
| Butylmethoxydibenzoylmethane | | | 1 | | 1 | |
| Sodium ascorbylphosphate | | | | | 0.2 | |
| Creatine | | | | 0.3 | | 0.5 |
| Creatinine | | | | 0.1 | | 0.5 |
| Ubiquinone (Q10) | | 0.05 | | 0.05 | | 0.02 |
| Taurine | | | 1.0 | | | |
| Phytic acid | | | | 0.1 | | |
| Tocopheryl acetate | | | 0.5 | | | |
| Tartaric acid, sodium salt | | | | | | 0.1 |
| α-Glucosylrutin | 0.1 | | | | | |
| Citric acid, sodium salt | 0.1 | | | | | |
| Glyceryl glucoside | 5 | 5 | 5 | 5 | 5 | 5 |
| Trisodium EDTA | | | 0.2 | | 0.2 | |
| Iminodisuccinate | | 0.1 | | | | |
| Phenoxyethanol | | 0.3 | | | 0.8 | 0.4 |
| p-Hydroxybenzoic acid alkyl ester (paraben) | | 0.5 | 0.9 | 0.3 | 0.4 | |
| Diazolidinylurea | | 0.25 | | | | 0.2 |
| Hexamidine diisethionate | | | | 0.040 | 0.05 | |
| 1,3-Dimethylol-5,5-dimethylhydantoin | | | 0.2 | | | |
| Iodopropynyl butylcarbamate | | 0.1 | | 0.05 | | |
| 2-Ethylhexyl glycerol ether | | | | | 3 | |
| Ethanol, denatured | 3 | 1 | 2 | | | 8 |
| Ammonium acryloyldimethyltaurate/VP copolymer | 0.3 | 0.7 | 0.5 | 0.2 | 0.1 | 0.8 |
| Glycerol | 10 | 8 | 10 | 5 | 15 | 5 |
| Butylene glycol | | | 1 | 2 | | |
| Fillers (distarch phosphate, SiO₂, talc, aluminium stearate) | 0.1 | 1 | 0.2 | 0.5 | 0.05 | 0.1 |
| Perfume/dyes | q.s. | q.s. | q.s. | q.s. | q.s. | q.s. |
| Water | ad 100 | ad 100 | ad 100 | ad 100 | ad 100 | ad 100 |

2. Preparation according to Claim 1, **characterized in that** the preparation comprises glucosyl glycerides in a total amount of from 0.01 to 10% by weight, based on the total weight of the preparation.

3. Preparation according to one of the preceding claims, **characterized in that** the preparation comprises powder raw materials in a total amount of from 0.01 to 10% by weight, based on the total weight of the preparation.

4. Preparation according to one of the preceding claims, **characterized in that** the powder raw materials have an average particle size of from 1 to 100 µm.

5. Cosmetic preparation according to one of the preceding claims, **characterized in that** the preparation is present in the form of a gel, an emulsion or a dispersion.

6. Cosmetic preparation according to one of the preceding claims, **characterized in that** the preparation comprises one or more UV filters selected from the group of the compounds phenylene-1,4-bis(2-benzimidazyl)-3,3'-5,5'-tetrasulphonic acid salts; 2-phenylbenzimidazole-5-sulphonic acid salts; 1,4-di(2-oxo-10-sulpho-3-bornylidenemethyl)benzene and salts thereof; 4-(2-oxo-3-bornylidenemethyl)benzenesulphonic acid salts; 2-methyl-5-(2-oxo-3-bornylidenemethyl)-sulphonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]-disiloxanyl]propyl]phenol; 3-(4-methylbenzylidene)-camphor; 3-benzylidenecamphor; ethylhexyl salicylate; terephthalidenedicamphorsulphonic acid; 2-ethylhexyl 4-(dimethylamino)benzoate; amyl 4-(dimethylamino)-benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; hexyl 2-(4'-diethylamino-2'-hydroxybenzoyl)benzoate, 4-(tert-butyl)-4'-methoxydibenzoylmethane; homomenthyl salicylate; 2-ethylhexyl 2-hydroxybenzoate; 2-ethylhexyl 2-cyano-3,3-diphenyl-acrylate; dimethicodiethyl benzalmalonate; 3-(4-(2,2-bis-ethoxycarbonylvinyl)phenoxy)propenyl)methoxy-silane/dimethylsiloxane copolymer; 2,4-bis{[4-(2-ethyl-hexyloxy)-2-hydroxy]phenyl}-6-(4-methoxyphenyl)-1,3,5-triazine (INCI: Bis-Ethylhexyloxyphenol Methoxyphenyl Triazine); dioctylbutylamidotriazone (INCI: Diethylhexyl-Butamidotriazone); 2,4-bis[5-1(dimethylpropyl)-benzoxazol-2-yl(4-phenyl)imino]-6-(2-ethylhexyl)imino-1,3,5-triazine with the CAS No. 288254-16-0; tris(2-ethylhexyl) 4,4',4"-(1,3,5-triazine-2,4,6-triyl-triimino)trisbenzoate (also: 2,4,6-tris[anilino(p-carbo-2'-ethyl-1'-hexyloxy)]-1,3,5-triazine (INCI: Ethylhexyl Triazone); 2,4,6-tribiphenyl-4-yl-1,3,5-triazine; merocyanines; titanium dioxide; zinc oxide in a concentration of from 0.01 to 40% by weight, based on the total weight of the preparation.

7. Cosmetic preparation according to one of the preceding claims, **characterized in that** the preparation comprises, as further ingredients, one or more compounds selected from the group consisting of folic acid, D-biotin, coenzyme Q10, alpha-glucosylrutin, carnitine, natural and/or synthetic isoflavonoids, genistein, flavonoids, carotenoids, creatine, creatinine, taurine, urea, ascorbic acid and derivatives, oxygen, tocopherol + esters, dihydroxyacetone; long-chain and short-chain hyaluronic acid (i.e. hyaluronic acid with an average molecular weight of 1 million to 3 million Daltons, such as also 5000 Daltons - 1 million Daltons); 8-hexadecene-1,16-dicarboxylic acid and/or licochalcone A.

8. Cosmetic preparation according to one of the preceding claims, **characterized in that** the preparation is present in the form of a O/W emulsion.

9. Cosmetic preparation according to one of the preceding claims, **characterized in that** the preparation comprises one or more O/W emulsifiers selected from the group of the compounds glyceryl stearate citrate, glyceryl stearate, cetearyl glucosides, stearic acid and its salts, polyglyceryl-3 methylglucose distearate, ceteareth-20, PEG-40 stearate, PEG-100 stearate, sodium cetearyl sulphate.

10. Cosmetic preparation according to one of the preceding claims, **characterized in that** the preparation according to the invention comprises one or more compounds selected from the group of parabens, phenoxyethanol, ethylhexylglycerol, 2-methylpropane-1,3-diol, butylene glycol, propylene glycol.

## Revendications

1. Préparation cosmétique contenant une association de
a) une ou plusieurs matières premières pour poudre, choisies dans le groupe de l'amidon de tapioca, de l'amidon d'octénylsuccinate sodique, de l'octénylsuccinate d'amidon et d'aluminium, du phosphate de diamidon et
b) un ou plusieurs glucosylglycérides,
**caractérisée en ce que** la préparation est exempte de (2-hydroxyéthyl)urée,
à l'exclusion des formules suivantes :
| Substance/% en poids | **1** | **2** | **3** | **4** | **5** | **6** |
|---|---|---|---|---|---|---|
| Stéarate-citrate de glycéryle | 2 | 1,5 | 3 | | | |
| PEG-40-stéarate | | | | | | 2 |
| Stéarate de glycéryle, autoémulsifiant | | | | 2 | | |
| Stéarate de glycéryle | | | | | 2 | 2 |
| Phosphate de cétyle | | | | | 0,5 | |
| Alcolanum | | 0,5 | | 0,5 | 0,5 | |
| Acide stéarique | | | | 2 | 3 | |
| Alcool myristylique | | | | 2 | 3 | |
| Alcool stéarylique | | 1 | | 0,5 | | 0,5 |
| Alcool cétylique | | | 3 | | | 2 |
| Alcool cétostéarylique | 3 | 1 | | 2 | | |
| Glycérides de graisse de coco hydrogénés | | 2 | | | 1 | |
| Dicaprylate/dicaprate de butylèneglycol | | 1 | | | | |
| Cocoate d'éthyle et d'hexyle | | 3 | | | | |
| Vaseline | | 4 | | | 2 | |
| Éther dicaprylylique | | 1 | 4 | | | |
| Myristate de myristyle | 3 | | | | 1,5 | |
| Beurre de karité | | | 2 | | | |
| Benzoates d'alkyle en C₁₂-C₁₅ | | | 3 | 3 | | 2 |
| Triglycérides d'acide caprylique/acide caprique | 4 | | 4 | | | 1 |
| Octyldodécanol | 1 | | | | | |
| Paraffinum Liquidum | | | 1 | | | |
| Cyclométhicone | | | 1 | 3 | 1 | 3 |
| Diméthicone | 1 | | | 1 | | |
| Polydécène | | | | | 1 | |
| Carbonate de dicaprylyle | | | | | | 2 |
| Méthoxycinnamate d'éthylhexyle | | 3 | 2 | | 5 | 3 |
| TiO₂ | | | | 1 | 1 | |
| 2-cyano-3-diphénylacrylate de 2-éthylhexyle | | | | 5 | | |
| Bis-éthylhexyloxyphénol-méthoxyphényltriazine | | 1 | | | | 1 |
| Éthylhexyltriazone | | | 2 | | | 2 |
| Butylméthoxydibenzoylméthane | | | 1 | | 1 | |
| Ascorbylphosphate de sodium | | | | | 0,2 | |
| Créatine | | | | 0,3 | | 0,5 |
| Créatinine | | | | 0,1 | | 0,5 |
| Ubiquinone (Q10) | | 0,05 | | 0,05 | | 0,02 |
| Taurine | | | 1,0 | | | |
| Acide phytique | | | | 0,1 | | |
| Acétate de tocophéryle | | | 0,5 | | | |
| Acide d-tartrique, sel de sodium | | | | | | 0,1 |
| α-glucosylrutine | 0,1 | | | | | |
| Acide citrique, sel de sodium | 0,1 | | | | | |
| Glycérylglucoside | 5 | 5 | 5 | 5 | 5 | 5 |
| EDTA trisodique | | | 0,2 | | 0,2 | |
| Iminodisuccinate | | 0,1 | | | | |
| Phénoxyéthanol | | 0,3 | | | 0,8 | 0,4 |
| p-hydroxybenzoate d'alkyle (parabène) | | 0,5 | 0,4 | 0,3 | 0,4 | |
| Diazolidinylurée | | 0.25 | | | | 0,2 |
| Hexamidine-diiséthionale | | | | 0,04 | 0,05 | |
| 1,3-diméthylol-5,5-diméthyl-hydantoïne | | | 0,2 | | | |
| Carbamate d'iodopropynylbutyle | | 0,1 | | 0,05 | | |
| 2-éthylhexylglycéroléther | | | | | 3 | |
| Éthanol dénaturé | 3 | 1 | 2 | | | 8 |
| Copolymère acryloyldiméthyl-taurate d'ammonium/VP | 0,3 | 0,7 | 0,5 | 0,2 | 0,1 | 0,8 |
| Glycérol | 10 | 8 | 10 | 5 | 15 | 5 |
| Butylèneglycol | | | 1 | 2 | | |
| Charges (phosphate de diamidon, SiO₂, talc, stéarate d'aluminium | 0,1 | 1 | 0,2 | 0,5 | 0,05 | 0,1 |
| Parfum/Colorants | q.s. | q.s. | q.s. | q.s. | q.s. | q.s. |
| Eau | à 100 | à 100 | à 100 | à 100 | à 100 | à 100 |

2. Préparation selon la revendication 1, **caractérisée en ce que** la préparation contient des glucosylglycérides en une quantité totale de 0,01 à 10 % en poids, par rapport au poids total de la préparation.

3. Préparation selon l'une quelconque des revendications précédentes, **caractérisée en ce que** la préparation contient des matières premières pour poudre en une quantité de 0,01 à 10 % en poids, par rapport au poids total de la préparation.

4. Préparation selon l'une quelconque des revendications précédentes, **caractérisée en ce que** les matières premières pour poudre présentent une taille moyenne de particule de 1 à 100 µm.

5. Préparation cosmétique selon l'une quelconque des revendications précédentes, **caractérisée en ce que** la préparation se trouve sous forme d'un gel, d'une émulsion ou d'une dispersion.

6. Préparation cosmétique selon l'une quelconque des revendications précédentes, **caractérisée en ce que** la préparation contient un ou plusieurs filtres UV choisis dans le groupe des composés sels d'acide phénylène-1,4-bis-(2-benzimidazyl)-3,3'-5,5'-tétrasulfonique ; sels d'acide 2-phénylbenzimidazole-5-sulfonique ; 1,4-di(2-oxo-10-sulfo-3-bornylidène-méthyl)-benzène et ses sels ; sels d'acide 4-(2-oxo-3-bornylidèneméthyl)-benzènesulfonique ; sels d'acide 2-méthyl-5-(2-oxo-3-bornylidèneméthyl)sulfonique ; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidènecamphre ; salicylate d'éthylhexyle ; acide téréphtalidène-dicamphosulfonique ; 4-(diméthylamino)-benzoate de 2-éthylhexyle ; 4-(diméthylamino)benzoate d'amyle ; 4-méthoxybenzalmalonate de di(2-éthylhexyle) ; 4-méthoxycinnamate de 2-éthylhexyle ; 4-méthoxycinnamate d'isoamyle ; 2-hydroxy-4-méthoxybenzophénone, 2-hydroxy-4-méthoxy-4'-méthylbenzophénone ; 2,2'-dihydroxy-4-méthoxybenzophénone ; 2-(4'-diéthylamino-2'-hydroxybenzoyl)-benzoate d'hexyle ; 4-(tert-butyl)-4'-méthoxydibenzoylméthane ; salicylate d'homo-menthyle ; 2-hydroxybenzoate de 2-éthylhexyle ; 2-cyano-3,3-diphénylacrylate de 2-éthylhexyle ; benzalmalonate de diméthicodiéthyle ; copolymère 3-(4-(2,2-bis-éthoxycarbonylvinyl)-phénoxy)propényl)-méthoxysiloxane / diméthylsiloxane ; 2,4-bis-{[4-(2-éthyl-hexyloxy)-2-hydroxy]-phényl}-6-(4-méthoxyphényl)-1,3,5-triazine (INCI : bis-éthylhexyloxyphénol méthoxyphényl triazine); dioctylbutylamidotriazone (INCI : diéthylhexyl-butamidotriazone) ; 2,4-bis-[5-1(diméthylpropyl)benzoxazol-2-yl-(4-phényl)-imino]-6-(2-éthylhexyl)-imino-1,3,5-triazine portant le n° CAS 288259-16-0 ; 4,4',4"-(1,3,5-triazine-2,4,6-triyltriimino)-tris-benzoate de tris(2-éthylhexyle) (également : 2,4,6-tris-[anilino-(p-carbo-2'-éthyl-1'-hexyloxy)]-1,3,5-triazine (INCI : éthylhexyl triazone) ; 2,4,6-tribiphényl-4-yl-1,3,5-triazine ; mérocyanine ; dioxyde de titane ; oxyde de zinc à une concentration de 0,01 à 40 % en poids par rapport au poids total de la préparation.

7. Préparation cosmétique selon l'une quelconque des revendications précédentes, **caractérisée en ce que** la préparation contient comme autres composants un ou plusieurs composés choisis dans le groupe constitué par l'acide folique, la D-biotine, la coenzyme Q10, l'alpha-glucosylrutine, la carnitine, les isoflavonoïdes naturels et/ou synthétiques, la génistéine, les flavonoïdes, les caroténoïdes, la créatine, la créatinine, la taurine, l'urée, l'acide ascorbique et ses dérivés, l'oxygène, le tocophérol + esters, la dihydroxyacétone ; un acide hyaluronique à longue chaîne ainsi qu'à courte chaîne (c'est-à-dire un acide hyaluronique ayant une masse moléculaire de 1 million à 3 millions de daltons, comme également 5 000 daltons - 1 million de daltons) ; l'acide 8-hexadécène-1,16-dicarboxylique et/ou la licochalcone A.

8. Préparation cosmétique selon l'une quelconque des revendications précédentes, **caractérisée en ce que** la préparation se trouve sous forme d'une émulsion H/E.

9. Préparation cosmétique selon l'une quelconque des revendications précédentes, **caractérisée en ce que** la préparation contient un ou plusieurs émulsifiants H/E choisis dans le groupe des composés stéarate-citrate de glycéryle, stéarate de glycéryle, cétéaryl-glucosides, acide stéarique ainsi que ses sels, 3-méthylglucosedistéarate de polyglycéryle, cétéareth-20, PEG-40 stéarate, PEG-100 stéarate, cétéarylsulfate de sodium.

10. Préparation cosmétique selon l'une quelconque des revendications précédentes, **caractérisée en ce que** la préparation selon l'invention contient un ou plusieurs composés choisis dans le groupe constitué par les parabènes, le phénoxyéthanol, l'éthylhexylglycérol, le 2-méthylpropane-1,3-diol, le butylèneglycol, le propylèneglycol.
